Europäisches Patentamt

European Patent Office

Office européen des brevets

(19)

(11) Publication number : **0 508 761 A2**

# (12) EUROPEAN PATENT APPLICATION

(21) Application number : **92303136.3**

(22) Date of filing : **08.04.92**

(51) Int. Cl.⁵ : **G01N 33/14**

(30) Priority : **10.04.91 GB 9107517**

(43) Date of publication of application :
**14.10.92 Bulletin 92/42**

(84) Designated Contracting States :
**BE DE ES GB NL**

(71) Applicant : **WHITBREAD PLC**
**Brewery, Chiswell Street**
**London EC1Y 4SD (GB)**

(72) Inventor : **Pyne, Nicholas Simon**
**14 Huskar Close, Silkstone**
**Nr Barnsley, South Yorkshire S75 4SX (GB)**
Inventor : **Grant, Andrew Philip**
**36 Honeysuckle Gardens**
**Hatfield, Hertfordshire, AL10 8PJ (GB)**
Inventor : **Ellicott, Michael Roy Teague**
**48 Letchworth Road**
**Limbury, Luton, Bedfordshire (GB)**

(74) Representative : **Wells, David**
**Gill Jennings & Every 53/64 Chancery Lane**
**London WC2A 1HN (GB)**

(54) **Measuring gas concentrations.**

(57)    A beverage, such as beer, is diverted from a main supply line (2) and passed through a mass-transfer cell (4). The cell (4) includes a gas permeable membrane (5), one side of which contacts the beverage. An inert carrier gas is passed through the cell (4) contacting the other side of the membrane (5). The carrier gas entrains head-promoting gas which has diffused across the member (5), and the gases are fed to a gas chromatograph and integrator (6) which produces an output suitable for controlling the concentration of the head-promoting gas to a desired level.

*Fig. 1.*

EP 0 508 761 A2

The present invention relates to a method and apparatus for treating a beverage to measure dissolved gas concentrations in the beverage, and in particular the measurement of nitrogen concentrations in a beverage such as beer.

It is commercially desirable that when beer is dispensed it should have a tight and stable head of foam on top. The carbon dioxide evolved from a carbonated beverage tends to produce a head of poor stability. It is found that the quality of the head can be markedly improved by introducing into the beer a small quantity of nitrogen or air. The effects of the introduction of such gases is however found to depend critically upon the precise nitrogen concentrations. If too little nitrogen is present then no significant improvement in the head is seen. In the presence of a relatively small excess of nitrogen, the beverage will tend to fob on being dispensed.

According to the present invention, a method of treating a beverage containing a head-promoting gas comprises diverting a portion of the flow from a main supply line carrying the beverage including the head-promoting gas through a mass-transfer cell including a gas permeable membrane one side of which in use is in contact with the beverage, passing an inert carrier gas through the mass-transfer cell, the inert carrier gas being in contact with the other side of the gas permeable membrane, the inert carrier gas thereby entraining head-promoting gas diffused across the membrane from the beverage, feeding the carrier gas with the entrained head-promoting gas into a gas chromatograph and integrator arranged to produce an output indicative of the concentration of the head-promoting gas in the beverage suitable for use in controlling the concentration to a desired level.

The present invention provides a method which enables on-line analysis of the dissolved gas levels so that the concentrations of the head-promoting gases can be controlled with far greater accuracy than hitherto has been possible, with a consequent increase in the quality and uniformity of the head produced when the beverage is dispensed.

Preferably the beverage flows continuously from the main supply line through the mass-transfer cell and back into the main supply line.

Preferably the method further comprises passing the beverage diverted from the main supply line through a heat exchanger to bring the beverage to a predetermined temperature prior to its introduction into the mass-transfer cell. Preferably the heat exchanger uses water as the heat transfer medium, and the method further comprises an initial calibration step of passing water from the heat exchanger into the mass transfer cell the head-promoting gas being present in the water in a known concentration and thereby providing a standard for the calibration of the gas chromatograph output.

According to a second aspect of the present invention, a system for treating a beverage comprises means for diverting at least part of the beverage carried through the main supply line through a mass-transfer cell, a gas permeable membrane arranged in the mass-transfer cell so that in use one side of the membrane comes in contact with the beverage, means for supplying an inert carrier gas to the mass-transfer cell, the inert carrier gas being in contact with the other side of the gas permeable membrane and entraining head-promoting gasses diffused across the membrane, and a gas chromatograph arranged to receive the inert carrier gas and the entrained head-promoting gasses and to provide an output indicative of the head-promoting gas concentration in the beverage.

Preferably the mass-transfer cell is connected to return the diverted beverage supply to the main supply line.

Preferably the carrier gas is supplied to the mass-transfer cell at a flow rate substantially less than the beverage flow rate, thereby enhancing the levels of the head-promoting gas in the sample supplied to the gas chromatograph.

A method and apparatus in accordance with the present invention will now be described in detail with reference to the accompanying drawings, in which:

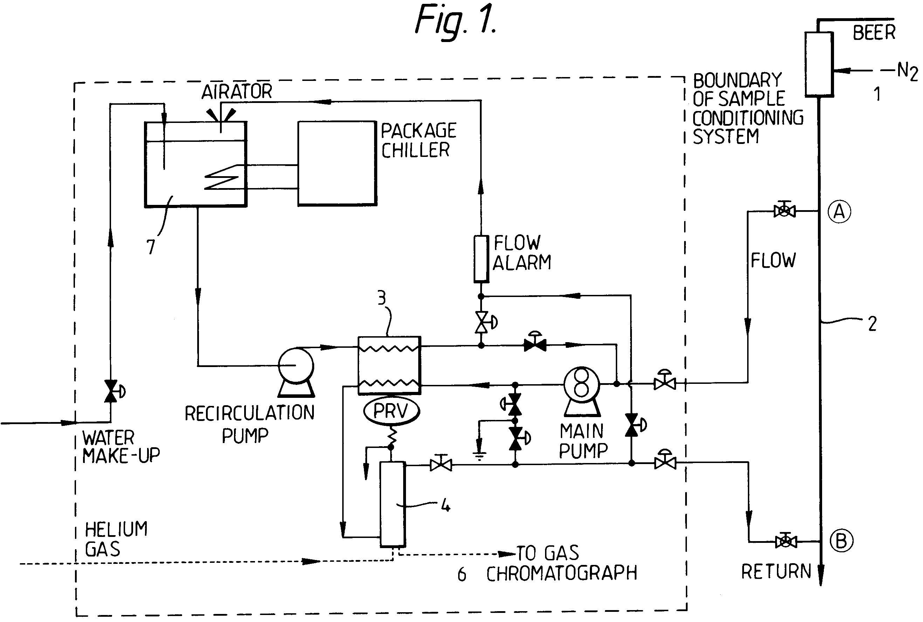

Figure 1 is a diagram showing the general arrangement of the beverage treating system;

Figure 2 is a sectional view of the mass-transfer cell; and

Figures 3 to 5 are graphs illustrating results obtained with the analyser of the preferred embodiment and with a prior art analyser.

A system for treating a beverage, which in the present example is beer, comprises a nitrogen introduction stage 1 and a beer main 2 which carries the beer together with the dissolved nitrogen on to a keg filling station (not shown). The nitrogen introduction stage may use any of a number of conventional techniques for the introduction of nitrogen, such as the use of a solution of nitrogen over a convolute flow path or alternatively use of a direct contact gas-liquid mixer.

At branch A in the beer main a proportion of the beer flowing through the main is diverted to flow through a heat exchanger 3 and a mass-transfer cell 4 before being reintroduced into the main supply downstream at point B.

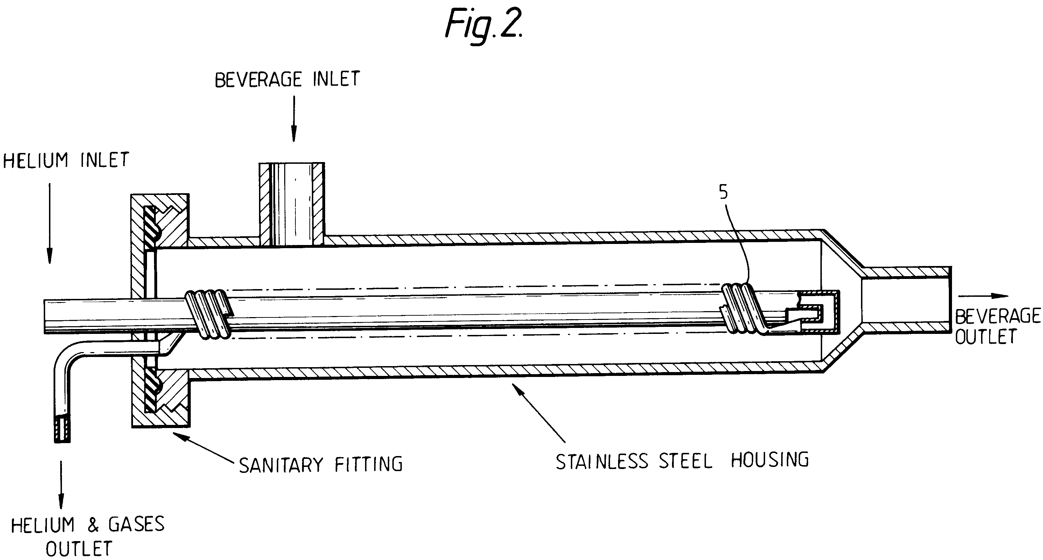

As shown in detail in Figure 2, the mass-transfer cell includes a gas permeable membrane in the form of a tube 5 extending into the cell 4. An inert carrier gas, which in the present example is helium, flows through the tube 5. The gas output from this supply is connected to a gas chromatograph 6 and integrator which in the present example is a Philips gas chromatograph fitted with a Shimadzu CRSA integrator. The gas in the tube may flow either in a counter-current or in a co-current direction. As an alternative to the use of a tube, a flat-plate permeable membrane may be used. Suitable membrane materials are silicon rubber or PTFE. As an alternative to helium, any other inert gas not normally present in the beverage and which will not have any effect on the beverage may be used, e.g. Argon.

In use, gases dissolved in the beer flowing through the cell diffuse through the membrane and are entrained by the flow of the inert carrier gas and so carried out to the gas chromatograph 6. The gas chromatograph 6 and integrator uses conventional analysis techniques to provide an indication of the gas concentrations present, and in particular of the concentration of nitrogen.

The heat exchanger 3 upstream of the mass-transfer cell 4 is used to bring the beer to a predetermined temperature. The use of a predetermined temperature and a set flow rate controlled by valves downstream of the mass-transfer cell ensures that accurate and reproducible results are obtained from the analysis. A flow of chilled water from a water cooling system 7 passes through a stainless steel heat exchanger in a counter-current direction. The heat exchanger can be any type suitable for controlling beverage temperatures and in this example is a shell and tube type. The water cooling and supply system 7 is arranged to ensure that the water is saturated with air at a known temperature. Then, since the gas concentrations including the nitrogen concentration in the water are precisely known, the supply from the system 7 is also used to calibrate the transfer cell and analysis system. Valves are provided to divert the flow of water from the system 7 through the mass-transfer cell. Nitrogen and other gasses then permeate through the membrane and are carried by the carrier to the gas chromatograph, thereby providing a known input which can be used for calibration. Subsequently when beer flows through the mass-transfer cell the results from the gas chromatograph are analysed and supplied to an external system for control of the nitrogen addition stage 1. This control may be effected automatically, or alternatively by manual intervention.

The results of trials carried out with the method and apparatus of this embodiment are described below.

## Example 1

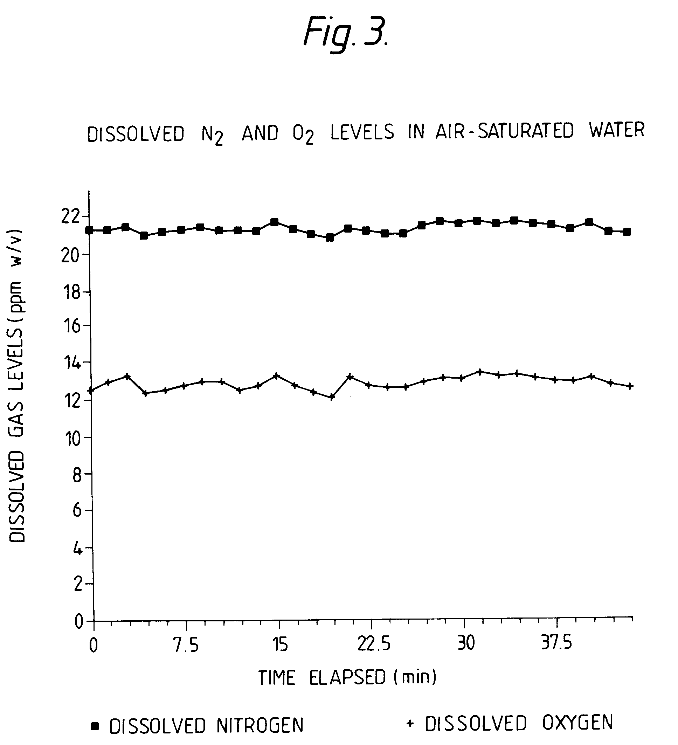

A production analyser was designed, built and installed on a beer line feeding a pasteuriser (in line heat exchanger allowing beer to be heated to a temperature of about 70°C held at that temperature and then cooled), within a racking facility (a facility for filling kegs with beer) at a brewery site. The analyser was calibrated using water saturated with air at 3°C. Then water saturated with air was fed into the analyser from the beer main. The information collected is displayed in Figure 3, for nitrogen and oxygen. This water contained 21.7 ppm nitrogen and 13.3 ppm oxygen. The analyser nitrogen output was accurate to ± 0.54 ppm.

## Example 2

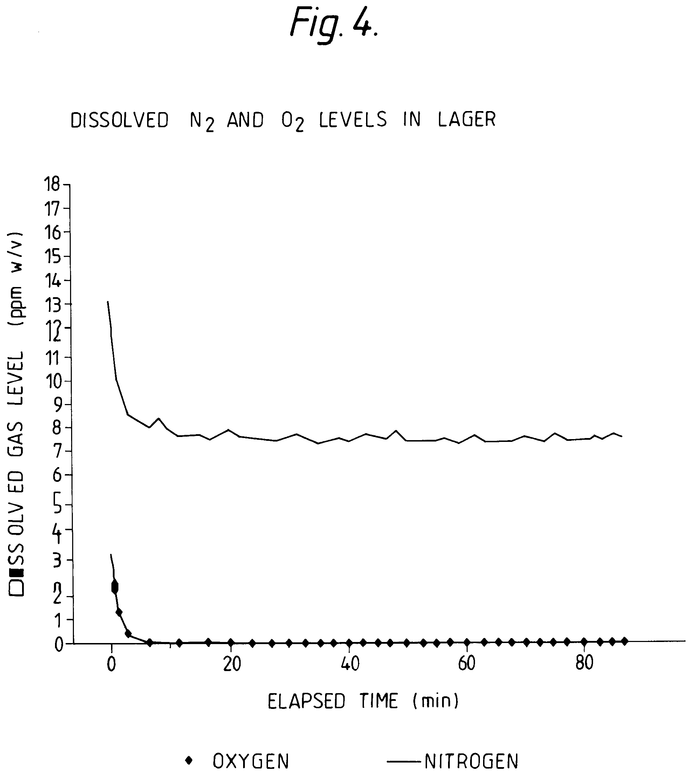

The feed to the analyser was changed from water and switched to a lager type beer. The data collected is shown in Figure 4. The change in reading over the first 5 minutes is the machine response to the change in nitrogen levels.

## Example 3

The feed was changed to a bitter beer and the analyser was moved so as to sample after the pasteuriser on the way to the racker. The beer flow in the main line was up to 200 hl/hour. The beer flow to the analyser was up to 10 litres/min but on average about 3 litres/min. The helium gas supply to the mass transfer cell was up to 100 ml/min at 10 psig and on average 10 ml/min. The temperature of the chilling water was controlled to 4°C (but could be 3 to 8°C). The gas sample exiting the mass transfer cell was injected onto the gas chromatography at intervals of just over 1 minute.

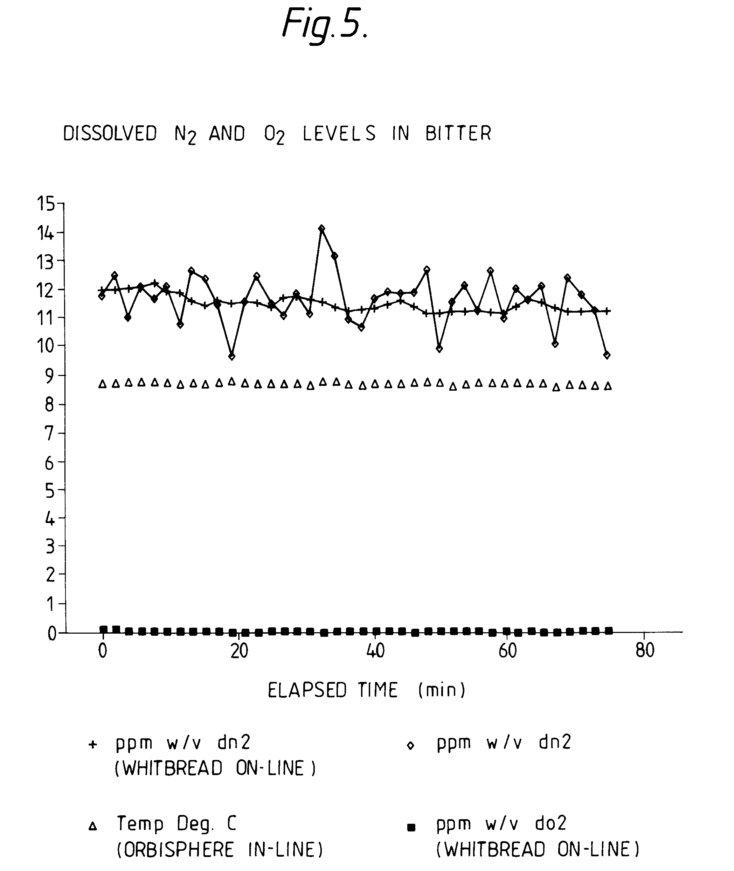

The results are displayed in Figure 5. The results obtained from the analyser of the present invention are plotted with crosses - + - and are seen to be highly stable. For comparison, results obtained with a prior art analyser (ORBISPHERE NITROGEN PROBE) are also plotted (points indicated by open diamond ( - ◊ ), and are seen to be highly erratic by comparison.

## Claims

1. A method of treating a beverage containing a head-promoting gas comprising diverting a portion of the flow from a main supply line (2) carrying the beverage including the head-promoting gas through a mass-transfer cell (4) including a gas permeable membrane (5) one side of which in use is in contact with the beverage, passing an inert carrier gas through the mass-transfer cell (4), the inert carrier gas being in contact with the other side of the gas permeable membrane (5), the inert carrier gas thereby entraining head-promoting gas diffused across the membrane from the beverage, feeding the carrier gas with the entrained head-promoting gas into a gas chromatograph (6) and integrator arranged to produce an output indicative of the concentration of the head-promoting gas in the beverage suitable for use in controlling the concentration to a desired level.

2. A method according to claim 1, in which the beverage flows continuously from the main supply line through the mass-transfer celt (4) and back into the main supply line (2).

3. A method according to claim 1 or 2, further comprising passing the beverage diverted from the main supply line through a heat exchanger (3) to bring the beverage to a predetermined temperature prior to its introduction into the mass-transfer cell.

4. A method according to claim 3, in which the heat exchanger (3) uses water as the heat transfer medium, the method further comprising an initial calibration step of passing water from the heat exchanger (3) into the mass transfer cell (4) the head-promoting gas being present in the water in a known concentration and thereby providing a standard for the calibration of the gas chromatograph output.

5. A method according to any preceding claim, in which the carrier gas is supplied to the mass-transfer cell (4) at a flow rate substantially less than the beverage flow rate, thereby enhancing the levels of the head-promoting gas in the sample supplied to the gas chromatograph.

6. A method according to any preceding claim, in which the flow rate of the beverage through the mass transfer cell is up to substantially 10 litres/min and the flow rate of the inert gas is up to substantially 100 ml/min.

7. A method according to claim 6, in which the flow rate of the beverage is substantially 3 litres/min and the flow rate of the inert gas is substantially 10 ml/min.

8. A method according to claim 4, or any one of claims 5 to 7 when dependent on claim 4, in which the temperature of chilled water in the heat exchanger is in the range from substantially 3°C to substantially 8°C.

9. A method according to claim 8, in which the temperature of the chilled water is substantially 4°C.

10. A method according to any preceding claim, in which the beverage is beer and the head-promoting gas is Nitrogen.

11. A system for treating a beverage comprising means for diverting at least part of the beverage carried through the main supply line (2) through a mass-transfer cell (4), a gas permeable membrane (5) arranged in the mass-transfer cell (4) so that in use one side of the membrane comes in contact with the beverage, means for supplying an inert carrier gas to the mass-transfer cell (4), the inert carrier gas being in contact with the other side of the gas permeable membrane (5) and entraining head-promoting gasses diffused across the membrane, and a gas chromatograph (6) arranged to receive the inert carrier gas and the entrained head-promoting gasses and to provide an output indicative of the head-promoting gas concentration in the beverage.

12. A system according to claim 11, in which the mass-transfer cell (4) is connected to return the diverted beverage supply to the main supply line (2).

13. A system according to claim 11 or 12, further comprising a heat exchanger (3) connected between the main arranged to bring the beverage to a predetermined temperature prior to its introduction into the mass-transfer cell.

14. A system according to claim 13, in which the heat exchanger (3) is arranged to use water as the heat transfer medium, the system further comprising means for supplying water from the heat exchanger to the mass-transfer cell (4) for calibration of the cell.

15. A method of treating a beverage comprising:
    introducing a head-promoting gas into the beverage;
    producing an output indicative of the gas concentration by a method according to any one of claims 1 to 10; and
    adjusting the amount of gas being introduced in accordance with the output.

Fig. 1.

EP 0 508 761 A2

*Fig.2.*

BEVERAGE INLET

HELIUM INLET

5

HELIUM & GASES
OUTLET

SANITARY FITTING

STAINLESS STEEL HOUSING

BEVERAGE
OUTLET

EP 0 508 761 A2

# Fig. 3.

DISSOLVED N$_2$ AND O$_2$ LEVELS IN AIR-SATURATED WATER

■ DISSOLVED NITROGEN          + DISSOLVED OXYGEN

# *Fig. 4.*

DISSOLVED N$_2$ AND O$_2$ LEVELS IN LAGER

* OXYGEN ——NITROGEN

# Fig.5.

DISSOLVED N$_2$ AND O$_2$ LEVELS IN BITTER

ELAPSED TIME (min)

+ ppm w/v dn2
(WHITBREAD ON-LINE)

⋄ ppm w/v dn2

△ Temp Deg. C
(ORBISPHERE IN-LINE)

■ ppm w/v do2
(WHITBREAD ON-LINE)